# EUROPEAN PATENT SPECIFICATION

(11) **EP 1 791 423 B1**
(45) Date of publication and mention of the grant of the patent: **01.05.2013**
(21) Application number: 04795060.5
(22) Date of filing: 14.10.2004
(51) Int. Cl.: A61K 31/704, A01N 65/00, A61K 38/00, A61K 45/00

(54) **COMPOSITIONS AND METHODS FOR THE MANAGEMENT OF HYPERPROLIFERATIVE DERMATOLOGICAL CONDITIONS**
ZUSAMMENSETZUNGEN UND VERFAHREN FÜR DIE BEHANDLUNG VON HYPERPROLIFERATIVEN HAUTKRANKHEITEN
COMPOSITIONS ET PROCEDES PERMETTANT DE TRAITER DES PROBLEMES DERMATOLOGIQUES SE CARACTERISANT PAR UNE HYPERPROLIFERATION

(30) Priority: 02.08.2004 US 710778
(43) Date of publication of application: 06.06.2007
(73) Proprietor: Sami Labs Limited, Bangalore 560 058 (IN)
(72) Inventor: MAJEED, Muhammed, Piscataway, NJ 08854 (US); PRAKASH, Subbalakshmi, Piscataway, NJ 08854 (US)
(74) Representative: Browne, Robin Forsythe
(86) International application number: PCT/US2004/033846
(87) International publication number: WO 2006/022762

(56) References cited:
- EP-A- 0 552 657
- WO-A-00/66111
- US-A- 3 670 084
- US-A- 5 536 506
- US-B1- 6 399 105
- PARNHAM M J ET AL: "Seleno-organic compounds and the therapy of hydroperoxide-linked pathological conditions" BIOCHEMICAL PHARMACOLOGY, PERGAMON, OXFORD, GB, vol. 36, no. 19, 1 October 1987 (1987-10-01), pages 3095-3102, XP025514476 ISSN: 0006-2952 [retrieved on 1987-10-01]
- THEROND P ET AL: "Toxicite du selenium a doses pharmacologiques par voie orale" NUTRITION CLINIQUE ET METABOLISME, ARNETTE EDITIONS, PARIS, FR, vol. 11, no. 2, 1 June 1997 (1997-06-01), pages 91-101, XP004884812 ISSN: 0985-0562
- SERWIN A B ET AL: "Selenium status in psoriasis" JEADV. JOURNAL OF THE EUROPEAN ACADEMY OF DERMATOLOGY AND VENEREOLOGY, ELSEVIER SCIENCE PUBLISHERS, AMSTERDAM, NL, vol. 11, 1 September 1998 (1998-09-01), page S296, XP004556184 ISSN: 0926-9959

## Description

### Technical field

The invention is related to compositions and methods for the management of hyperproliferative skin conditions such as psoriasis, and their associated symptoms. The compositions described herein contain a natural leukotriene inhibitor selected from Boswellia serrata gum resin, its extractives, isolates or derivatives in combination with a bioavailable organic selenium nutritional supplement. These compositions are administered orally and topically to the individual human or animal in need of treatment at optimal levels and in suitable dosage forms to manifest the desired benefits, with no untoward side effects.

### Background art

Psoriasis is a chronic skin disease marked by periodic flare-ups of sharply defined red patches covered by a silvery, flaky surface. In psoriasis the skin epidermis is subjected to destructive changes due to genetic abnormalities in the immune system that are triggered by stress and environmental factors. There is hyperproliferation of immature keratinocytes in the epidermis, forming patches or plaques with silvery, flaky dead skin shed from the surface. In the dermis, the blood vessels provide an increased blood supply to the abnormally multiplying keratinocytes, causing the underlying inflammation and redness characteristic of psoriasis. Several types of psoriasis exist occurring independently, concurrently or consequently. The most common form of psoriasis is plaque psoriasis. Psoriatic arthritis is a debilitating disorder that includes both psoriasis and arthritis.

Psoriasis is clinically addressed with topical medications, phototherapy, systemic medications. Agents such as steroidal drugs, cyclosporine, Methothrexate, oral and topical retinoids, selenium sulfide used in pharmaceutical preparations for this purpose have various side effects. There is no known cure for psoriasis.

Boswellia serrata (Roxb.) Burseraceae is a large tree which grows in the dry and hilly parts of India. The exudate of Boswellia serrata is a gum resin commonly known as "Dhup", "Indian Frankincense"or "Indian Olibanum"(Anonymous, 1948 and Chopra et al, 1956), The gum resin known as Salai Guggal has been used in Ayurvedic system of medicine for the treatment of rheumatism, respiratory diseases and liver disorders (Kirtikar and Basu, 1935). Ancient Ayurvedic texts (Shushrutha Samhitha Gulabkunvarba,1949) and Charaka Samhitha (Chowkhamba,1968) mentioned the anti-rheumatic activity of guggals. The nonacidic oil fraction of gum resins of Boswellia was found to exhibit significant pain relieving effects in rats (Menon and Kar, 1971 and Menon and Kar, 1969). The acidic fraction of gum Boswellia serrata, containing boswellic acids, was found to exhibit antipyretic action in rats and rabbits (Singh and Atal, 1986, Singh et al., 1993). The acidic fraction of gum Boswellia serrata, however, was not known to have any analgesic property. In nature, boswellic acids occur in the gum or exudate from the tree Boswellia serrata (Roxb.). The gum contains a mixture of four boswellic acids.

Boswellic acids inhibit 5-lipoxygenase (Safayhi et al., 1992) the enzyme which catalyzes conversion of arachidonic acid to inflammatory leukotrienes. Boswellic acids also inhibit the enzyme human leukocyte elastase (HLE) which catalyzes connective tissue break down (Safayhi et al., 1997).

Inflammatory leukotrienes have been implicated in the pathogenesis and pathophysiology of psoriasis. In lesional skin from psoriasis and eczema patients, leukotriene B4 was found to increase by approximately 6.6-fold (Reilly et al. 2000). A 5-lipoxygenase catalyzed product from arachidonic acid, leukotriene B4, is considered to play a significant role in the pathogenesis of psoriasis (Iversen et al., 1997). A massive increase in human leukocyte elastase (HLE) activity was found in lesional skin of psoriasis (31 times), allergic contact dermatitis (55 times), and atopic dermatitis (35 times), but not in uninvolved skin of diseased patients (Wiedow, 1992).

WO 96/19212 describes the use of boswellic acids in treating brain tumors.

WO 97/07796 describes the use of boswellic acid and its derivatives for inhibiting normal and increased leukocytic elastase or plasmin activity.

US 5,888,514 describes a natural composition containing boswellic acids for treating bone or joint inflammation.

US 5,629,351 describes a boswellic acid composition and the preparation thereof.

US 5,494,668 describes a boswellic acid composition for treating musculoskeletal disorders.

US 5,720,975 describes the use of incense for treating Alzheimer"s disease.

EP 0552657 describes the use of pure boswellic acid, a physiologically acceptable salt, derivative, or a salt of the derivative or a boswellic acid containing herbal preparation for the prophylaxis and/or control of inflammatory processes caused by increased leukotriene formation in human and veterinary medicine.

WO 00/57893 describes compositions containing Boswellia serrata extract wherein Boswellia serrata extract, boswellic acid or a derivative thereof is useful in preparing skin care or hair care compositions with the ability to provide a soothing effect to irritated skin

Selenium is a vital trace element nutrient with multiple roles in the growth and functioning of living cells in higher animals and humans. At the molecular level, selenium (as selenocysteine) is an essential component of the active sites of the antioxidant enzyme glutathione peroxidase, and the enzymes participating in thyroid functions iodothyronine-5'-deiodinase and mammalian thioredoxin reductase. Selenium is also present in several other mammalian selenoproteins. Low selenium status has been linked with the occurrence of decreased immunity to diseases and the prevalence of various forms of cancer.

Patients with moderate or severe psoriasis have been shown to have low blood selenium levels and increased levels of malondialdehyde, a product of free radical induced oxidation (Corrocher, et al. 1989). Low selenium dietary intake has been linked to the progression of psoriasis in a clinical study (Serwin, et al. 1999). It has been demonstrated in both human subjects and experimental animals that topical selenomethionine reduces the degree of damage to the skin induced by UV radiation (Burke, et al., 1992). The antioxidant action of selenomethionine through its role in the antioxidant enzymes (Cronin, 2000) may therefore help in cases of psoriasis.

Recent studies suggest that that psoriasis is not primarily a skin disorder but an immunological disturbance under the skin. The skin manifestations are a result of overstimulation of superficial skin cells (Langerhans cells) due to increased production of interleukin 2, 6 and 8 as well the diminished production of transforming growth-factor-alpha Interleukin-10 (Christ, 1999)

The effect of Selenium supplementation (400 micrograms/day for 6 weeks as Se-yeast, containing about 70% selenomethionine) on skin and blood Se-content, on skin glutathione peroxidase activity and on various chemical and immunological parameters of blood and skin was investigated in 7 psoriatic patients. The results of this study suggested that selenomethionine may be able to modulate the immunological mechanism of psoriatic lesions by increasing the number of CD4+ T cells (Harvima, 1993).

Tumor necrosis factor-alpha (TNF-alpha) and its receptors play important roles in the induction and maintenance of psoriatic lesions. A recent study reports that oral supplementation with selenomethionine was ineffective as adjuvant treatment in plaque psoriasis and may contribute to the maintenance of elevated TNF-R1 (soluble TNF-alpha receptor type 1) concentration in psoriasis patients despite the remission of skin lesions. (Serwin et al., 2003)

Patent application CN1233482 describes the application of glossy selenium enriched ganoderma in the treatment of psoriasis.

WO02096429 describes an agent for the external therapy of psoriasis in the form of liposomal emulsions of preparations of methylxathine group, selenium and some other preparations are used as active substances.

IT1244459 describes Pharmaceutical compositions which can be administered topically for the treatment of skin diseases such as vitiligo, acne, psoriasis, alopecia, hypotrichosis, comprising one or more oils of animal origin selected from cod liver oil, mink oil and tortoise oil containing lithium, zinc, copper and possibly gold, silver, sulphur, selenium and silicon dissolved or dispersed in them are described.

United States Patent: 6,630,442 describes a composition of glutathione and selenium, as a selenoamino acid or selenium yeast extract and an epidermal growth factor in a topical carrier and method of using the composition to reduce and repair skin damage, resulting from aesthetic (exfoliation and chemical peels) and surgical (laser and other therapies) procedures and other chemical and thermal burns to the cutaneous tissues.

None of the prior art cited above addresses psoriasis with a natural dual inhibitor of 5-lipoxygenase and human leukocyte elastase in combination with organic selenium supplement. The anti-complementary activity of boswellic acids is detailed in literature (Kapil, A et al. 1992). The complement-dependent induction of TNF-alpha is a well-established pathway. Therefore the combination treatment of the current invention would prevent any potential elevation of TNF-alpha levels not addressed by selenium supplementation.

Anonymous (1948), The Wealth of India: Raw Materials, Vol I, CSIR Publications, Delhi, pp 208- 210.

Chopra, R.N. Nayar, S.L., Chopra, I.C. (1956), Glossary of India Medicinal Plants, SCIR, Delhi.Chowkhamba (1968), Charaka Samhita (2nd ed), Sanskrit Series Office, Varanasi.

Kirtikar, K.R. and Basu, B.D. (1935), Indian Medicinal Plants, Vol. I, pp. 521- 529.

Menon, M.K. and Kar. A. (1971), Analgesic and psycho-pharmacological effects of the gum resin of Boswellia serrata. Planta Med. 19:338 - 341.

Menon, M.K. and Kar, A. (1969). Analgesic effects of the gum resin of Boswellia serrata. Life Sciences 8(1):1023-28.

Singh, G.B. and Atal, C.K. (1986), Pharmacology of an extract of salai guggal ex-Boswellia serrata, a new non-steroidal anti-inflammatory agent, Agents and Actions 18 (3/4):407- 411.

Singh, G.B. et al. (1993), Boswellic Acids, Drugs of the Future 18(4):307 309.

Sushruta Samhita (1949), Shree Gulabkunvarba, Vol 1 6, Ayurvedic Soc., Jamnagar.

Reilly DM. et al. (2000) Inflammatory mediators in normal, sensitive and diseased skin types. Acta Derm Venereol 80(3):171-4

Iversen L, et al. (1997) Significance of leukotriene-A4 hydrolase in the pathogenesis of psoriasis. Skin Pharmacol. 10(4):169-77

Wiedow O, et al. (1992) Lesional elastase activity in psoriasis, contact dermatitis, and atopic dermatitis. Invest Dermatol. 99(3):306-9

Safayhi, H. et al. (1992) Boswellic acids: novel, specific, non-redox inhibitors of 5-lipoxygenase. J. Pharmacol. Exp. Ther. 261:1143-6.

Safayhi, H. et al. (1997) Inhibition by boswellic acids of human leukocyte elastase. J. Pharmacol. Exp. Ther. 281:460-463

Corrocher, R. et al. (1989) Effect of fish oil supplementation on erythrocyte lipid pattern, malondialdehyde production and glutathione-peroxidase activity in psoriasis. Clin Chim Acta 179(2):121-31

Serwin AB, et al. (1999) Selenium nutritional status and the course of psoriasis. Pol Merkuriusz Lek. 6(35):263-5

Burke, K.E. et al. (1992) The effect of topical L-selenomethionine on minimal erythema dose of ultraviolet irradiation in humans. Photodermatol Photoimmunol Photomed 9(2):52-7

Cronin, J.R. (2000) Dietary selenium: Elemental Nutrition for Muscles, Immunity, Well-Being and Cancer Prevention. Alt. Complement. Therap. 6(6):342-346.

Christ HW. (1999) Immunomodulating therapy of psoriasis vulgaris. Med Klin 94 Suppl 3:90-2

Serwin, A.B. et al. Soluble tumor necrosis factor-alpha receptor type 1 during selenium supplementation in psoriasis patients. Nutrition. 2003 Oct;19(10):847-850.

Harvima, R.J. (1993) Screening of effects of selenomethionine-enriched yeast supplementation on various immunological and chemical parameters of skin and blood in psoriatic patients. Acta Derm. Venerol. 73(2): 88-91.

Kapil, A and Moza, N Anticomplementary activity of boswellic acids-an inhibitor of C3-convertase of the classical complement pathway. Int J Immunopharmacol. 1992 Oct;14(7):1139-43.

### Disclosure of the Invention

Hyperproliferative skin disorders are exemplified by psoriasis. Psoriasis is a chronic skin disease characterized by periodic flare-ups in the form of red patches covered by a silvery, flaky surface that appear on the skin. It is attributed to genetic abnormalities in the immune system that are triggered by environmental factors, and therefore classified as an autoimmune disorder. There are several variants of psoriasis of which plaque psoriasis is the most common and is seen on the elbows, knees and lower back. In psoriatic arthritis, psoriatic lesions are accompanied by symptoms of arthritis. Psoriatic arthritis is characterized by stiff, tender, and inflamed joints.

The current invention addresses hyperproliferative skin conditions with a novel treatment regimen in which a natural dual acting inhibitor of 5-lipoxygenase and human leukocyte elastase is ingested orally, as well as applied topically, in combination with a bioavailable organic selenium supplement which is ingested orally

The processes leading to all autoimmune disease involve the human leukocyte antigen (HLA) system, which is genetically regulated. Malfunction of this system is at the root of most immune disorders, including psoriatic arthritis. The current invention is directed towards supplying nutrients that would potentially inhibit the inflammatory process through inhibiting the expression of pro-inflammatory enzymes and modulating the expression of keratinocyte growth factor. Successful treatment entails configuring the correct dosing regimen that would favorably influence the underlying genetic processes involved.

### Brief Description of Drawings

Figure 1 shows the appearance of psoriatic lesions in patient 1, patient 2, patient 3 initially, at the end of week 8 (visit 1) and the end of week 12 (visit 2) during treatment.

Figure 2 shows the appearance of psoriatic lesions in patient 4, patient 5, patient 6 initially, at the end of week 8 (visit 1) and the end of week 12 (visit 2) during treatment.

Figure 3 shows the appearance of psoriatic lesions in patient 7, patient 8, patient 9 initially, at the end of week 8 (visit 1) and the end of week 12 (visit 2) during treatment.

Figure 4 shows the appearance of psoriatic lesions in patient 10, patient 11, patient 12 initially, at the end of week 8 (visit 1) and the end of week 12 (visit 2) during treatment.

### Mode(s) for carrying out the invention

Exemplary embodiments of the current invention present compositions and dosing regimens to effect successful treatment of psoriasis.

**Example1 Compositions of the Invention**

An exemplary embodiment of the active ingredient composition of the oral formulation of the invention is presented in Table 1.

**Table 1: Active Ingredient Composition of the Oral dosage form**

| **Active ingredient** | **Quantity** |
|---|---|
| Beta-boswellic acid | 64mg |
| Acetyl-beta-boswellic acid | 48mg |
| 11-keto-beta-boswellic acid | 24mg |
| Acetyl-11-keto-beta-boswellic acid | 32mg |
| L(+)-Selenomethionine (trituration in Dicalcium phosphate) | 20mg |

The formulation is prepared in the form of a capsule, tablet, power, spansule or other dosage form for oral administration, with commonly used excipients and additives for such purposes.

An exemplary embodiment of the formulation for topical application is presented in Table 2.

**Table 2: Composition of the topical cream**

| **Ingredient** | **% (w/w)** |
|---|---|
| Purified water | 60.000 |
| Carbom er 940 | 0.250 |
| Glycerin | 4.000 |
| Methylparaben | 0.200 |
| Edetate sodium | 0.010 |
| Cetyl alcohol | 3.500 |
| Stearyl alcohol | 3.500 |
| Stearic acid | 6.500 |
| Glyceryl stearate | 2.500 |
| PEG-100 stearate | 2.500 |
| Isopropyl palmitate | 6.000 |
| Vitamin E acetate | 1.000 |
| Dimethicone | 0.100 |
| Propylparaben | 0.100 |
| Vitamin A Palmitate | 0.100 |
| Ascorbyl palmitate | 0.200 |
| Boswellin® | 5.000 |
| Purified water | 2.000 |
| Triethanolamine | 0.400 |
| Imiduria . | 0.300 |

Boswellin (a trademark of Sabinsa Corporation, NJ, USA) is a standardized Boswellia serrata gum resin extract containing beta-boswellic acid , acetyl-beta-boswellic acid , 11-keto-beta-boswellic acid and Acetyl-11-keto-beta-boswellic acid.

The active ingredient may be formulated as a cream, lotion, patch, gel or any other topical dosage form.

Example 2 Treatment of psoriatic human subjects with the composition and method of the invention

12 patients (6 males and 1 female) 18-65 years of age, presenting with primary plaque and secondary scale manifested as annular, polycyclic, morbiliform lesions accompanied by Itching, peeling of skin, and bleeding on scratching, participated in the study.

The patients was administered tablets containing 400 mg boswellic acids and 100 mcg of selenium in the form of L(+)-Selenomethionine orally and also treated with a topical cream containing 400 mg of boswellic acids.

The daily dose regimen followed was 1 tablet as above with topical application as above, thrice daily for 150 days. PASI score (Psoriasis Area Severity Index) which takes into account body surface area as well as erythema (redness), induration (thickness), and scaliness, was assessed at 4 week intervals. Serum biochemical parameters were similarly assessed.

Psoriasis affected parts of the body were photographed at the initiation of the study and at 4 week intervals

The patients were followed up for another 30 days and showed improvement in symptoms of psoriasis with no untoward side effects.

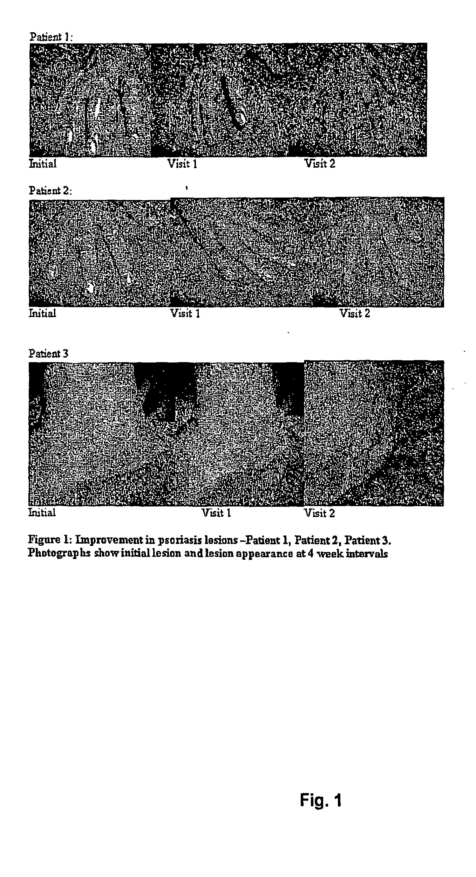

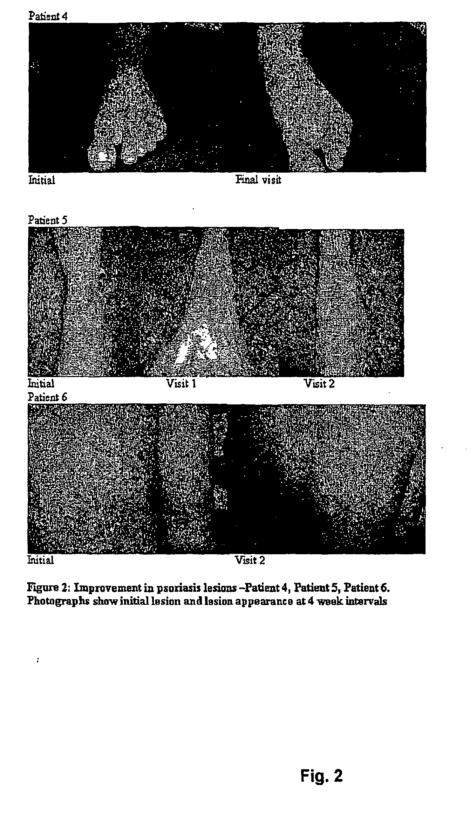

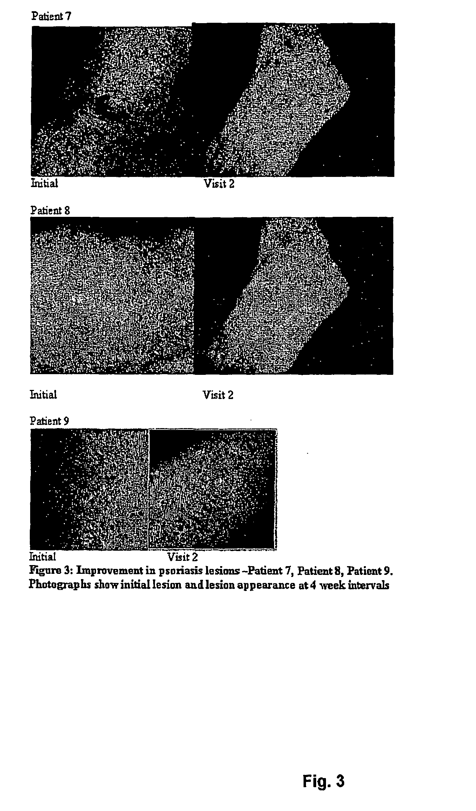

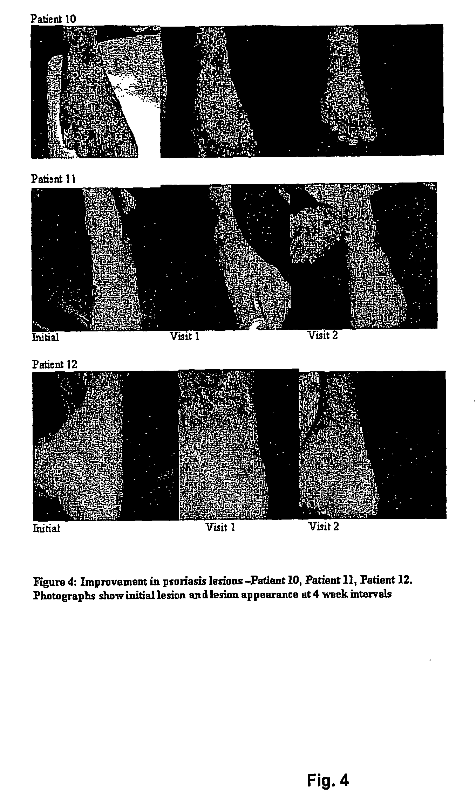

The effects of the treatment on the external manifestations of psoriasis are photographically represented in Figures 1-4.

## Claims

1. Use of a composition for manufacturing a medicament for treating a psoriasis affected area of skin, scalp or nails, wherein the composition comprises:
(a) an orally administered formulation comprising boswellic acid and a bioavailable organic selenium, dispersed in a pharmaceutically acceptable carrier; and
(b) a topically applied formulation of boswellic acid dispersed in a pharmaceutically acceptable carrier.

2. Use of a medicant as claimed in claim 1, wherein the orally administered formulation contains from about 50 mg to about 500 mg of boswellic acid represented by one or more of following groups:
(a) beta-boswellic acid, acetyl beta-boswellic acid, 11-keto beta-boswellic acid, acetyl-11-keta-beta-boswellic acid, as single entities or mixtures;
(b) congeners or derivatives of beta-boswellic acid, acetyl beta-boswellic acid, 11-keto beta-boswellic acid, acetyl-11-keto-beta-boswellic acid, as single entities or mixtures.

3. Use of a medicant as claimed in claim 1 or 2, wherein the orally administered formulation contains 100 micrograms of selenium derived from one or more of following selenium sources: selenomethionine, Se-methylselenocysteine, derivatives of selenomethionine, derivatives of Se-methylsclcnocysteine and high selenium yeast.

4. Use of a medicant as claimed in any preceding claim, wherein the topically administered formulation contains from about 50 mg to about 400 mg of boswellic acid represented by one or more of following groups:
(a) beta-boswellic acid, acetyl beta-boswellic acid, 11-keto beta-boswellic acid, acetyl-11-keto-beta-boswellic acid, as single entities or mixtures;
(b) congeners or derivatives of beta-boswellic acid, acetyl beta-boswellic acid, 11-keto beta-boswellic acid, acetyl-11-keto-beta-boswellic acid, as single entities or mixtures.

5. Use of a medicant as claimed in any preceding claim, wherein the orally administered formulation contains from about 10 mg to about 400 mg of acetyl-11-keto-beta-boswellic acid.

6. Use of a medicant as claimed in any preceding claim, wherein the orally administered formulation contains from about 10 micrograms to about 100 micrograms of L (+)-selenomethionine, dispersed in a pharmaceutically acceptable carrier.

7. Use of a medicant as claimed in any preceding claim, wherein the topically administered formulation contains from about 10 mg to about 400 mg of acetyl-11-keto-beta-boswellic acid, dispersed in a pharmaceutically acceptable carrier.

8. Use of a medicant as claimed in any preceding claim, wherein the oral dose of boswellic acid is 400 mg administered thrice daily.

9. Use ofa medicant as claimed in any preceding claim, wherein the total daily oral dose of selenium is from about 10 micrograms to about 400 micrograms.

10. Use of a medicant as claimed in any preceding claim, wherein the topical dose of boswellic acid is 400 mg administered thrice daily.

11. A composition comprising
(a) an oral formulation comprising boswellic acid and bioavailable organic selenium, dispersed in a pharmaceutically acceptable carrier; and
(b) a topical formulation comprising boswellic acid dispersed in a pharmaceutically acceptable carrier.

12. The composition as claimed in claim 11, wherein the boswellic acid is at an amount of 400 mg and the bioavailable organic selenium is at an amount of 100 micrograms.

## Patentansprüche

1. Verwendung einer Zusammensetzung für die Herstellung eines Medikamentes zur Behandlung von Psoriasis, die Bereiche der Haut, der Kopfhaut oder der Nägel befallen hat, wobei die Zusammensetzung folgendes aufweist:
(a) eine Zubereitung für die orale Verabreichung, die Boswelliasäure und ein biologisch verfügbares organisches Selen aufweist, die in einem pharmazeutisch akzeptablen Träger dispergiert sind; und
(b) eine Zubereitung für die topische Anwendung von Boswelliasäure, die in einem pharmazeutisch akzeptablen Träger dispergiert ist.

2. Verwendung eines Medikamentes nach Anspruch 1,
wobei die Zubereitung für die orale Verabreichung etwa 50 mg bis etwa 500 mg Boswelliasäure enthält, die mit einer oder mehreren der folgenden Gruppen angegeben wird:
(a) β-Boswelliasäure, Acetyl-β-boswelliasäure, 11-keto-β-Boswclliasäure, Acetyl-11-keto-β-boswelliasäure, als einzelne Bestandteile oder Gemische;
(b) gleichartige Verbindungen oder Derivate von β-Boswelliasäure, Acetyl-β-boswelliasäure, 11-keto-β-Boswelliasäure, Acetyl-11-keto-β-boswelliasäure, als einzelne Bestandteile oder Gemische.

3. Verwendung eines Medikamentes nach Anspruch 1 oder 2,
wobei die Zubereitung für die orale Verabreichung 100 µg Selen enthält, das aus einer oder mehreren der folgenden Selenquellen stammt: Selenomethionin, Se-methylselenocystein, Derivate von Selenomethionin, Derivate von Se-methylselenocystein und selenreiche Hefe.

4. Verwendung eines Medikamentes nach einem vorstehenden Ansprüche, wobei die Zubereitung für die topische Anwendung etwa 50 mg bis etwa 400 mg Boswelliasäure enthält, die mit einer oder mehreren der folgenden Gruppen angegeben wird:
(a) β-Boswelliasäure, Acetyl-β-boswelliasäure, 11-keto-β-Boswelliasäure, Acetyl-11-keto-β-boswelliasäure, als einzelne Bestandteile oder Gemische;
(b) gleichartige Verbindungen oder Derivate von β-Boswelliasäure, Acetyl-β-boswelliasäure, 11-keto-β-Boswelliasäure,
Acetyl-11-keto-β-boswelliasäure, als einzelne Bestandteile oder Gemische.

5. Verwendung eines Medikamentes nach einem der vorstehenden Ansprüche, wobei die Zubereitung für die orale Verabreichung etwa 10 bis etwa 400 mg Acetyl-11-keto-β-boswelliasäure enthält.

6. Verwendung eines Medikamentes nach einem der vorstehenden Ansprüche, wobei die Zubereitung für die orale Verabreichung et wa 10 bis etwa 100 µg L(+)-Selenomethionin, in einem pharmazeutisch akzeptablen Träger dispergiert, enthält.

7. Verwendung eines Medikamentes nach einem der vorstehenden Ansprüche, wobei die Zubereitung für die topische Verabreichung etwa 10 mg bis etwa 400 mg Acetyl-11-keto-β-boswelliasäure, in einem pharmazeutisch akzeptablen Träger dispergiert, enthält.

8. Verwendung eines Medikamentes nach einem der vorstehenden Ansprüche, wobei die orale Dosis von Boswelliasäure 400 mg, dreimal täglich verabreicht, beträgt.

9. Verwendung eines Medikamentes nach einem der vorstehenden Ansprüche, wobei die gesamte tägliche orale Dosis von Selen etwa 10 µg bis etwa 400 µg beträgt.

10. Verwendung eines Medikamentes nach einem der vorstehenden Ansprüche, wobei die topische Dosis von Boswelliasäure 400 mg, dreimal täglich verabreicht, beträgt.

11. Zusammensetzung, die folgendes aufweist:
(a) eine orale Zubereitung, die Boswelliasäure und biologisch verfügbares organisches Selen aufweist, die in einem pharmazeutisch akzeptablen Träger dispergiert sind; und
(b) eine topische Zubereitung, die Boswelliasäurc aufweist, die in einem pharmazeutisch akzeptablen Träger dispergiert ist.

12. Zusammensetzung nach Anspruch 11,
wobei die Boswelliasäure in einer Menge von 400 mg und das biologisch verfügbare organische Selen in einer Menge von 100 µg vorliegen.

## Revendications

1. Utilisation d'une composition pour la fabrication d'un médicament destiné au traitement d'une zone de la peau, du cuir chevelu ou des ongles affectée par un psoriasis, laquelle composition comprend :
a) une formulation à administrer par voie orale, comprenant de l'acide boswellique et du sélénium organique biodisponible, dispersés dans un véhicule pharmacologiquement admissible,
b) et une formulation à administrer en topique, comprenant de l'acide boswellique dispersé dans un véhicule pharmacologiquement admissible.

2. Utilisation d'une composition médicinale conforme à la revendication 1, dans laquelle la formulation à administrer par voie orale contient d'environ 50 mg à environ 500 mg d'acide boswellique, constitué par l'un ou plusieurs des ensembles suivants :
a) acide bêta-boswellique, acide acétyl-bëta-boswellique, acide 11-céto-bêta-boswellique et acide acétyl-11-céto-bêta-boswellique, seuls ou mélangés ;
b) composés voisins ou dérivés de l'acide bêta-boswellique, de l'acide acétyl-bêta-boswellique, de l'acide 11-céto-bêta-boswellique et de l'acide acétyl-11-céto-bêta-boswellique, seuls ou mélangés.

3. Utilisation d'une composition médicinale conforme à la revendication 1 ou 2, dans laquelle la formulation à administrer par voie orale contient environ 100 µg de sélénium, dérivé de l'une ou de plusieurs des sources suivantes de sélénium : sélénométhionine, Se-méthyl-sélénocystéine, dérivés de sélénométhionine, dérivés de Se-méthyl-sélénocystéine, et levure enrichie en sélénium.

4. Utilisation d'une composition médicinale conforme à l'une des revendications précédentes, dans laquelle la formulation à administrer en topique contient d'environ 50 mg à environ 400 mg d'acide boswellique, constitué par l'un ou plusieurs des ensembles suivants :
a) acide bêta-boswellique, acide acétyl-bêta-boswellique, acide 11-céto-bêta-boswellique et acide acétyl-11-céto-bêta-boswellique, seuls ou mélangés ;
b) composés voisins ou dérivés de l'acide bêta-boswellique, de l'acide acétyl-bêta-boswellique, de l'acide 11-céto-bêta-boswellique et de l'acide acétyl-11-céto-bêta-boswellique, seuls ou mélangés.

5. Utilisation d'une composition médicinale conforme à l'une des revendications précédentes, dans laquelle la formulation à administrer par voie orale contient d'environ 10 mg à environ 400 mg d'acide acétyl-11-céto-bêta-boswellique.

6. Utilisation d'une composition médicinale conforme à l'une des revendications précédentes, dans laquelle la formulation à administrer par voie orale contient d'environ 10 µg à environ 100 µg de L(+)-séléno-méthionine, dispersée dans un véhicule pharmacologiquement admissible.

7. Utilisation d'une composition médicinale conforme à l'une des revendications précédentes, dans laquelle la formulation à administrer en topique contient d'environ 10 mg à environ 400 mg d'acide acétyl-11-céto-bêta-boswellique, dispersé dans un véhicule pharmacologiquement admissible.

8. Utilisation d'une composition médicinale conforme à l'une des revendications précédentes, dans laquelle la dose d'acide boswellique prise par voie orale vaut 400 mg, administrée trois fois par jour.

9. Utilisation d'une composition médicinale conforme à l'une des revendications précédentes, dans laquelle la dose quotidienne totale de sélénium prise par voie orale vaut d'environ 10 µg à environ 400 µg.

10. Utilisation d'une composition médicinale conforme à l'une des revendications précédentes, dans laquelle la dose d'acide boswellique prise en topique vaut 400 mg, administrée trois fois par jour.

11. Composition comprenant :
a) une formulation à administrer par voie orale, comprenant de l'acide boswellique et du sélénium organique biodisponible, dispersés dans un véhicule pharmacologiquement admissible,
b) et une formulation à administrer en topique, comprenant de l'acide boswellique dispersé dans un véhicule pharmacologiquement admissible.

12. Composition conforme à la revendication 11, dans laquelle l'acide boswellique se trouve en une quantité de 400 mg et le sélénium organique biodisponible se trouve en une quantité de 100 µg.
